# EUROPÄISCHE PATENTANMELDUNG

(11) **EP 4 574 983 A1**
(43) Veröffentlichungstag der Anmeldung: **25.06.2025**
(21) Anmeldenummer: 23219719.4
(22) Anmeldetag: 22.12.2023
(51) Int. Cl.: C12P 7/18, C12P 19/02, C12P 19/24

(54) **VERFAHREN ZUR HERSTELLUNG VON ALLITOL**

(71) Anmelder: Annikki GmbH, 8074 Raaba-Grambach (AT)
(72) Erfinder: Staunig, Nicole, 8074 Raaba-Grambach (AT); Dupont, Maria, 8074 Raaba-Grambach (AT)
(74) Vertreter: Schwarz & Partner Patentanwälte GmbH

(57) **Zusammenfassung**

Verfahren zur Herstellung von Allitol, indem aus D-Fructose, welche in einer wässerigen Lösung gelöst vorliegt, durch Behandeln mit einer Epimerase *in vitro* D-Psicose gebildet wird, welche durch Behandeln mit einer NAD(P)H-abhängigen Oxidoreduktase *in vitro* zu Allitol reduziert wird, wobei das durch die Reduktion entstandene NAD(P)' mit einem Wasserstoff-Donor enzymatisch wieder zu NAD(P)H reduziert wird, dadurch gekennzeichnet, dass als Wasserstoff-Donor ein sekundärer Alkohol eingesetzt wird.

## Beschreibung

Die vorliegende Erfindung betrifft ein enzymatisches Verfahren zur Herstellung des seltenen Zuckeralkohols Allitol aus D-Fructose.

### Hintergrund der Erfindung

Allitol ist ein in der Natur selten vorkommender sechswertiger Zuckeralkohol, der beispielsweise in den Blättern von Rosmarinweiden (*Itea* sp.) nachgewiesen werden konnte (Hough & Stacey, 1963). Er ist achiral und bildet daher eine Schnittstelle zwischen den D- und L-Hexosen in der sogenannten Izumoring-Strategie (Izumori, 2006; Hassanin et al., 2017). Allitol kann daher als Vorstufe zur Herstellung von D-Psicose (Gullapalli et al., 2007; Poonperm et al., 2007) oder L-Psicose (Takeshita et al., 1996) dienen. Darüber hinaus kann Allitol aufgrund seines süßen Geschmacks auch als Süßungsmittel eingesetzt werden (Hassanin et al., 2017). WO 2020195106 A1 beschreibt eine Einsatzmöglichkeit von Allitol als Antiadipositum (Schlankheitsmittel).

Allitol ist von D-Fructose aus in zwei chemischen Umsetzungsschritten erreichbar: 1) Epimerisierung von D-Fructose zu D-Psicose (C3-Epimer) und 2) Reduktion von D-Psicose zu Allitol.

Der erste Schritt wird mit einer Ketose-3-Epimerase bewerkstelligt (Izumori et al., 1993). Ketose-3-Epimerasen lassen sich je nach Substratspezifität in drei Gruppen einteilen: 1) D-Tagatose-3-Epimerase (DTE), 2) D-Psicose-3-Epimerase (DPE) oder D-Allulose-3-Epimerase (DAE) und 3) L-Ribulose-3-Epimerase (LRE). Bei der Epimerisierung bildet sich ein Gleichgewichtsverhältnis zwischen beiden Epimeren aus. Je nach Reaktionsbedingungen (Temperatur zwischen 40 und 70 °C, pH-Wert zwischen 6 und 11) liegt dieses zwischen 80:20 und 62,5:37,5 (D-Fructose : D-Psicose). Viele der Epimerasen benötigen außerdem ein zweiwertiges Metallion wie Mn²⁺ oder Co²⁺ (toxisch) als Kofaktor (Zhang et al., 2016; Jiang et al., 2020).

Die Reduktion von D-Psicose zu Allitol kann z.B. mikrobiell mit *Enterobacter agglomerans* Stamm 221e (Muniruzzaman et al., 1995) oder mit *Klebsiella oxytoca* G4A4 (Han et al., 2014) durchgeführt werden.

Die NAD-abhängige Ribitol-Dehydrogenase (RDH; EC 1.1.1.56), die für die Umwandlung des fünfwertigen Zuckeralkohols Ribitol zur Ketopentose D-Ribulose verantwortlich ist, katalysiert ebenfalls die Reduktion von D-Psicose zu Allitol.

Zhu et al. (2015) exprimierten D-Psicose-3-Epimerase aus *Ruminococcus* sp. und RDH aus *Klebsiella oxytoca,* eine Formiat-Dehydrogenase (FDH) aus *Candida methylica* (zur Regeneration des Kofaktors Nicotinamidadenindinukleotid NADH; oxidiert Formiat zu CO₂) sowie ein Glucose-Fructose-Facilitator-Genprodukt aus *Zymomonas mobilis* in *Escherichia coli.* Mit diesen Modifikationen konnte der rekombinante Stamm 16,5 g/l Allitol aus 18 g/l D-Fructose (92% Umsatz nach 18 h) produzieren.

Wen et al. (2022) nutzten ebenfalls einen rekombinanten E. coli-Stamm (mit exprimierter RDH und FDH), der 58,5 g/l Allitol aus 90 g/l D-Psicose in 1 h produzierte.

Wang et al. (2023) beschreiben einen *E*. coli-Ganzzell-Biokatalysator zur Umwandlung von D-Fructose zu Allitol. Die verwendeten *E.* coli-Zellen beinhalteten eine DPE aus *Clostridiales,* eine RDH aus *Providencia alcalifaciens,* eine FDH aus *Starkeya* sowie eine weitere DPE aus *Rhizobium straminoryzae.* Auf diese Weise wurde D-Fructose (500 mM = 90 g/l) innerhalb von 12 h bei 37 °C und pH 6 unter Einsatz von 1000 mM Natrium-Formiat (zwei Äquivalente bezogen auf D-Fructose) und 0,5 mM NAD⁺ zu 452 mM Allitol umwandelt (Umsatz 90,4%). Als Nebenprodukt entstand ca. 30 mM D-Sorbitol (das Reduktionsprodukt der D-Fructose). Die Zellen wurden durch Zentrifugation abgetrennt und ausgetretene Proteine im Allitol-haltigen Überstand wurden durch Hitzeeinwirkung deaktiviert.

Die Verwendung einer FDH zur Kofaktor-Regeneration weist einige Nachteile auf: 1. Bildung von klimaschädlichem CO₂ als Oxidationsprodukt von Formiat, 2. Verschiebung des pH-Werts der Reaktion in den basischen Bereich (Neuhauser et al., 1998; Kratzer et al., 2015), 3. Produktion großer Mengen an Abfall bestehend aus nicht umgesetztem Natrium-Formiat und Natriumsulfat (bei Verwendung von Schwefelsäure zur pH-Kontrolle) sowie 4. die geringe spezifische Aktivität von Formiat-Dehydrogenasen (Boldt & Ansorge-Schumacher, 2020; Tishkov & Popov, 2004).

Ein alternatives häufig eingesetztes enzymatisches Kofaktor-Regenerationssystem ist die Glucose-Dehydrogenase (GDH) mit D-Glucose als Substrat, welches mittels NAD(P)⁺ zu D-Gluconolacton oxidiert wird, wobei NAD(P)H gebildet wird. Das Lacton hydrolysiert im wässerigen Milieu zu D-Gluconsäure/D-Gluconat. Im Vergleich zur FDH entsteht bei der Kofaktor-Regeneration mittels GDH kein klimaschädliches CO₂ als Nebenprodukt.

Zhao et al. (2022) verwendeten ein Multienzym-Selbstassemblierungssystem aus DPE, RDH und Glucose-Dehydrogenase (GDH; zur Kofaktor-Regeneration) in Kombination mit einer Glucose-Isomerase in einem Ganzzell-System, um Allitol (15 g/l) direkt aus D-Glucose (25 g/l) herzustellen.

Feng et al. (2023) beschreiben ein *in* vivo-Verfahren mit einem *E.* coli-Ganzzellkatalysator mit exprimierter DPE aus *Clostridium bolteae* (erfordert den Zusatz von 1 mM Co²⁺), GDH aus *B. subtilis,* sowie RDH aus *Providencia alcalifaciens* zur gleichzeitigen Produktion von Allitol und D-Gluconsäure aus D-Fructose und D-Glucose. Selbst mit einem *E*. coli-Ganzzellkatalysator, der unter optimierten Fermentationsbedingungen erhalten wurde, konnten nur 10,6 g/l D-Gluconsäure aus 25 g/l D-Glucose sowie 9,8 g/l Allitol aus 25 g/l D-Fructose innerhalb von 12 h hergestellt werden, was von den Autoren als "unwirtschaftlich" bezeichnet wurde. Weitere Experimente mit unterschiedlichen Konzentrationen von D-Glucose (bei gleichbleibender D-Fructose-Konzentration von 25 g/l) zeigten, dass die höchsten Umsätze (14,8 g/l D-Gluconsäure und 12 g/l Allitol) mit 20 g/l D-Glucose am Start der Reaktion erzielt werden können. Das bedeutet, dass mindestens 1/5 der eingesetzten D-Fructose nicht zu Allitol umgesetzt werden kann. In weiterer Folge verwendeten Feng et al. Zuckerrohr-Melassen, welche zur Hydrolyse von Saccharose mittels Schwefelsäure und Ultraschall vorbehandelt wurden, als Substrat. Innerhalb von 12 h konnten 42,7 g/l Allitol (Umsatz 30,7%) und 56,2 g/l D-Gluconsäure (Umsatz 37,7%) aus 139,2 g/l D-Fructose und 149,1 g/l D-Glucose erhalten werden, was der Meinung der Autoren nach auf die zu geringe Menge an Biomasse zurückzuführen sein könnte. Zur Trennung der Mischung aus Produkten und Substraten werden neben der Kristallisation (zum Teil kostspielige und aufwendige) Methoden wie Membran-Trennverfahren wie Nanofiltration oder auch Simulated Moving Bed Chromatography (SMBC) vorgeschlagen.

Zur Herstellung von Allitol sind auch zellfreie Verfahren beschrieben.

Hassanin et al. (2016) nutzten eine Ribitol-Dehydrogenase (RDH) aus *Providencia alcalifaciens* RIMD 1656011 und eine FDH aus *Ogataea parapolymorpha* DL-1 in Form von Zell-Lysaten zum Umsatz von D-Psicose (10 g/l) zu Allitol (94% in 6 h; Zusatz von 2 mM NAD⁺). Zur Gewinnung von Allitol wurde eine HPLC-Methode verwendet - das gesammelte Eluat wurde gefriergetrocknet.

Die Regenerierung von Kofaktoren mittels Alkoholdehydrogenasen ist z.B. aus der EP 2812439 B1 vorbekannt oder in Xu et al. (2021) beschrieben.

Takeshita et al. (2000) setzen eine Formiat-Dehydrogenase (FDH) zur Regenerierung des Kofaktors ein. Es wird eine DTE aus *Pseudomonas cichorii* ST-24 und eine RDH aus *Klebsiella pneumoniae* Stamm X22 (eine Mutante von *K. pneumoniae* IFO 3321) in Form von Zell-Lysaten zur Umsetzung von D-Fructose (10 g/l) unter Zusatz von 2,5 mM NAD⁺ in 48 h zu Allitol (100% Umsatz) eingesetzt. Reines Allitol wurde durch die Behandlung der Reaktionsmischung mit Aktivkohle, Zentrifugation/Filtration gefolgt von Deionisation mittels lonentauscherharzen aus der Produktlösung gewonnen. In Takeshita et al. (2000) wird ferner eine Optimierung des Verfahrens zur Herstellung von Allitol aus D-Fructose beschrieben. Ein Nachteil dieses Verfahrens besteht trotz Optimierung aber immer noch darin, dass D-Fructose nur in relativ niedrigen Konzentrationen von etwa 10 g/l umgesetzt werden kann.

Hier setzt nun die Aufgabe der vorliegenden Erfindung an und setzt sich zum Ziel, ein Verfahren zur Herstellung von Allitol zur Verfügung zu stellen, welches die oben genannten Verfahren von Takeshita et al. (2000) sowie Feng et al. (2023) verbessert und welches insbesondere gestattet, D-Fructose in höheren Substratkonzentrationen einzusetzen und zusätzlich höhere Umsätze zu erzielen.

### Detaillierte Beschreibung der Erfindung

Die Aufgabe wird erfindungsgemäß gelöst, indem aus D-Fructose, welche in einer wässerigen Lösung vorliegt, durch Behandeln mit einer Epimerase *in vitro* D-Psicose gebildet wird, die durch Behandeln mit einer NAD(P)H-abhängigen Oxidoreduktase *in vitro* zu Allitol reduziert wird, wobei das bei der Reduktion entstandene NAD(P)⁺ mit einem Wasserstoff-Donor enzymatisch wieder zu NAD(P)H reduziert wird, und ist dadurch gekennzeichnet, dass als Wasserstoff-Donor ein sekundärer Alkohol eingesetzt wird.

Ein entscheidendes Merkmal der vorliegenden Erfindung besteht darin, dass das Verfahren *in vitro* ausgeführt wird, d.h. nicht fermentativ.

Ein sekundärer Alkohol ist im Sinne der vorliegenden Beschreibung und Patentansprüche eine organische Verbindung mit einer sekundären Alkoholgruppe.

Es hat sich gezeigt, dass bei erfindungsgemäßem Einsatz eines sekundären Alkohols als Wasserstoff-Donor - anstatt von Ameisensäure (Formiat) als Wasserstoff-Donor - D-Fructose in einer viel höheren Substratkonzentration eingesetzt werden kann als im Stand der Technik.

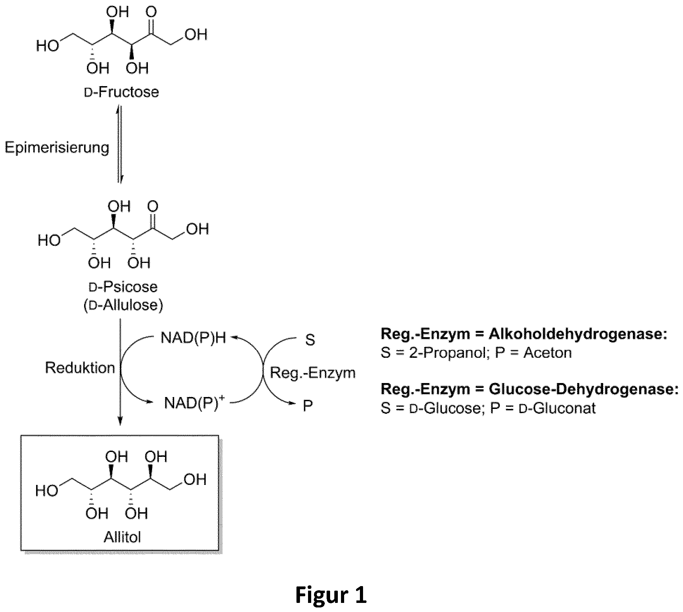

Im ersten Schritt wird aus D-Fructose durch Behandeln mit einer Epimerase *in vitro* D-Psicose gebildet, welches mit einer NAD(P)H-abhängigen Oxidoreduktase *in vitro* zu Allitol reduziert wird. Allitol kann im Anschluss durch Kristallisation aus der Lösung abgetrennt werden. Das erfindungsgemäße Verfahren ist in der beiliegenden Figur 1 schematisch dargestellt.

Eine bevorzugte Variante des erfindungsgemäßen Verfahrens besteht darin, dass der sekundäre Alkohol 2-Propanol (Isopropanol) ist. 2-Propanol ist ein sehr günstiger Wasserstoff-Donor zur Regeneration von NAD(P)H und das Oxidationsprodukt Aceton ist aufgrund seiner Flüchtigkeit leicht abtrennbar (Xu et al., 2021). Aus dem Abgasstrom gewonnenes Aceton kann heterogen-katalytisch wieder zu 2-Propanol hydriert werden (Al-Rabiah et al., 2022), entweder in der Gasphase, in Lösung oder in Isopropanol/Aceton/Wasser Mischungen, wobei in Zukunft vermehrt auf Wasserstoff aus nachhaltigen Quellen ("grüner Wasserstoff") gesetzt werden könnte. Des Weiteren kann die Reduktion von Aceton zu 2-Propanol auch enzymatisch mittels Alkoholdehydrogenase (in Kombination mit einer enzymatischen Oxidation) bewerkstelligt werden.

Eine weitere bevorzugte Variante des erfindungsgemäßen Verfahrens besteht darin, dass der durch die Reduktion von D-Psicose zu Allitol entstandene oxidierte Kofaktor NAD(P)⁺ mittels einer Glucose-Dehydrogenase (GDH) und D-Glucose unter Bildung von D-Gluconolacton reduziert wird, welches je nach pH-Wert im wässerigen Milieu zu D-Gluconsäure oder D-Gluconat hydrolysiert. D-Gluconsäure ist aufgrund ihrer vielfältigen Anwendungsmöglichkeiten wie beispielsweise als Metallbeizmittel (D-Gluconsäure), Säuerungsmittel (D-Gluconsäure und D-Gluconolacton), Entrostungsmittel (Natriumgluconat) oder Nahrungsergänzungsmittel (Calciumgluconat, Magnesiumgluconat, Eisengluconat) eine wichtige Industriechemikalie (Kornecki et al., 2020; EP 0132557 B1).

Die Verwendung der oben beschriebenen Kofaktor-Regenerationssysteme (D-Glucose + GDH sowie 2-Propanol + ADH) vermeidet die Emission von klimaschädlichem CO₂ und darüber hinaus große Abfallmengen (wie nicht umgesetztes Natrium-Formiat beim FDH-Regenerationssystem), da die Produkte zum einen weiteren Anwendungen zugeführt werden können (D-Gluconat) und zum anderen chemisch-katalytisch (Hydrierung) oder enzymatisch regeneriert werden können (Aceton zu 2-Propanol).

Eine weitere bevorzugte Variante des erfindungsgemäßen Verfahrens ist dadurch gekennzeichnet, dass es als Eintopf-Reaktion ohne Isolierung von Zwischenprodukten durchgeführt wird.

Die besonders bevorzugte Konzentration von D-Fructose beträgt 50 - 250 g/l.

Die besonders bevorzugte Konzentration von D-Glucose beträgt 50 - 250 g/l.

Der besonders bevorzugte Temperaturbereich für das erfindungsgemäße Verfahren liegt zwischen 25 und 45 °C.

Der besonders bevorzugte pH-Bereich liegt zwischen 7 und 8,5.

Bei einer weiteren, bevorzugten Variante des erfindungsgemäßen Verfahrens liegen die Enzyme in einer Suspension und/oder im Homogenat und/oder im Lysat der entsprechenden, sie bildenden Zellen vor, wobei Lysate besonders bevorzugt sind.

Suspension bedeutet im Sinne der vorliegenden Beschreibung und Patentansprüche eine Suspension von *resting cells.* Diese werden nach der Kultivierung geerntet (vom Nährmedium abgetrennt) und in einem geeigneten Puffersystem suspendiert. Im Gegensatz zu fermentativen Verfahren, bei denen auch mit ganzen Zellen gearbeitet wird, können die *resting cells* aufgrund der Entfernung von Kohlenstoff-Quellen und Nährstoffen nicht mehr wachsen, sondern dienen nur der Umsetzung von Substraten (Lin & Tao, 2017). Homogenat steht in diesem Kontext für eine physikalisch und/oder chemisch behandelte Suspension (z.B. mittels Druckes, Lysozym oder Ultraschall behandelt), wobei die Zellbestandteile aus den Zellen freigesetzt werden. Ein Lysat wird erhalten, wenn die unlöslichen Zellbestandteile des Homogenats beispielsweise durch Filtration oder Zentrifugation entfernt werden (siehe *Produktion der Enzyme* & *Herstellung der Lysate* für Details).

In einer weiteren Variante können die Enzyme auch mit einem wasserlöslichen Polymer wie Polyethylenglycol am N-Terminus modifiziert, in oder auf einer festen Matrix immobilisiert oder Teil eines Fusionsproteins sein.

In einer weiteren Variante können die Enzyme in Pulverform, in lyophilisierter oder sprühgetrockneter Form vorliegen.

In einer besonders bevorzugten Ausführung des Verfahrens werden für die Konversion des Startmaterials nur Enzyme aus den Enzymgruppen Epimerasen und Oxidoreduktasen verwendet, wobei eines oder mehrere dieser Enzyme aus jeder dieser Gruppen ausgewählt werden.

Die im Verfahren verwendete Epimerase kann aus einer der Gruppen EC 5.1.3.30 (D-Psicose-3-Epimerase) oder EC 5.1.3.31 (D-Tagatose-3-Epimerase/L-Ribulose-3-Epimerase) stammen, wobei die erstgenannte besonders bevorzugt ist.

Das zur Reduktion von D-Psicose verwendete Enzym stammt aus der Gruppe der Oxidoreduktasen, wobei eine Short-Chain-Dehydrogenase/Reduktase besonders bevorzugt ist.

Die NAD(P)H-abhängige Oxidoreduktase zur Reduktion von D-Psicose zu Allitol umfasst oder besteht vorzugsweise aus einer Aminosäuresequenz, welche ausgewählt ist aus der Gruppe bestehend aus:
i) einer Aminosäuresequenz, die eine Identität zu SEQ ID Nr. 2 von mindestens 80% aufweist,
ii) einer Aminosäuresequenz, die durch eine Nukleinsäure kodiert wird, die eine Identität zu SEQ ID Nr. 1 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 1 bindet.

Besonders geeignet zur Reduktion von D-Psicose zu Allitol im Allgemeinen ist eine Oxidoreduktase, deren Aminosäuresequenz mindestens 80% ident zu SEQ ID Nr. 2 ist bzw. welche durch eine Nukleinsäure kodiert wird, die eine Identität zu SEQ ID Nr. 1 von mindestens 80% aufweist bzw. unter stringenten Bedingungen an ein Nukleinsäuremolekül mit der Nukleinsäuresequenz SEQ ID Nr. 1 bindet.
SEQ ID Nr. 1: 
ATGACACAGTCTCTTCAGGGCAAGATCGTCGCCATTACTGGCGCGGCTTCGGGCATTGGCCTCGAATGC
GCCCGCTATCTCATCGAAGCTGGCGCGGTGGTCTATCTTCTGGACCGTGACGCCAAAACTCTCGAAGAC
AAAACGGCAGAACTCGGCAGCCAGGCCCATGCGATCATCGTCGATCTCTTCGACTACAAAACCGTAGAT
 
GCTGCGGTCGCGCAGATCGTTGAGGAGCAGGGCCGGATCGATGTTTTCCACGCCAACGCCGGCGCGTA
TGTGGGCGGCAATGTCTGGGAAGGGGATCCCGATAGCTGGGATGCGATGCTGCACCTGAACATCAAT
GCAGCTTTCCGCTCGGTCCGTGCCGTGCTGCCGCAGATGATGAAGCAGGAAAGTGGCGATATCGTCAT
GACCAGCTCGATCGCGGGCATGATCCCCATCATGGCCGAGCCGATCTACACGGCCTCGAAACATGCCG
TGCAGGCATTCGTACATACGGTGCGCCGACAGGTCGCGAAGTATGGAATTCGTGTGGGTGCAATCCAG
CCTGGTCCTGTAGTCACGCCTTTGCTGAAAGACTGGGATCAGGCCCGTCTTGAAGCCAACATCAAAGCG
GGTGCCCTGATGGAAGCCAAGGAAGTCGCCGAAGCCCTGATCTTCATCCTGACACGTTCAAAGGGTGT
CATGGTGCGGGATCTGGTCGTTCTGCCACATAACTTCGACGCCTAAGCTTAAGCGGCCGCACTCGAGCA
CCACCACCACCACCACTGAGATCCGGCTGCTAA
SEQ ID Nr. 2:

MTQSLQGKIVAITGAASGIGLECARYLIEAGAVVYLLDRDAKTLEDKTAELGSQAHAIIVDLFDYKTVDAAVA
QIVEEQGRIDVFHANAGAYVGGNVWEGDPDSWDAMLHLNINAAFRSVRAVLPQMMKQESGDIVMTSSI
AGMIPIMAEPIYTASKHAVQAFVHTVRRQVAKYGIRVGAIQPGPVVTPLLKDWDQARLEANIKAGALMEA
KEVAEALIFILTRSKGVMVRDLVVLPHNFDA

Die hier angeführte Oxidoreduktase zur Reduktion von D-Psicose zu Allitol umfasst oder besteht vorzugsweise aus einer Aminosäuresequenz, die eine Identität zu SEQ ID Nr. 2 von mindestens 80%, noch mehr bevorzugt von 85%, noch mehr bevorzugt von 90%, noch mehr bevorzugt von 95%, noch mehr bevorzugt von 98%, noch mehr bevorzugt von 99%, insbesondere von 100%, aufweist. Besonders bevorzugt umfasst die erfindungsgemäße Oxidoreduktase zur Reduktion von D-Psicose zu Allitol die Aminosäuresequenz SEQ ID Nr. 2 oder besteht aus dieser.

Alternativ umfasst oder besteht die Oxidoreduktase zur Reduktion von D-Psicose zu Allitol vorzugsweise aus einer Aminosäuresequenz, die durch eine Nukleinsäure kodiert wird, die eine Identität zu SEQ ID Nr. 1 von mindestens 80%, noch mehr bevorzugt von 85%, noch mehr bevorzugt von 90%, noch mehr bevorzugt von 95%, noch mehr bevorzugt von 98%, noch mehr bevorzugt von 99%, insbesondere von 100%, aufweist. Besonders bevorzugt umfasst die Nukleinsäure, welche die erfindungsgemäße Oxidoreduktase zur Reduktion von D-Psicose zu Allitol kodiert, die Nukleinsäuresequenz SEQ ID Nr. 1 oder besteht aus dieser.

Der Begriff "Identität", wie hier verwendet, bezieht sich auf den Prozentsatz der identischen Nukleotid- bzw. Aminosäureübereinstimmungen zwischen mindestens zwei, unter Verwendung eines standardisierten Algorithmus miteinander ausgerichteten Nukleotid- bzw. Aminosäuresequenzen ("Alignment"). Ein solcher Algorithmus kann, in einer standardisierten und reproduzierbaren Weise, Lücken ("Gap") in die verglichenen Sequenzen einfügen, um das Alignment zwischen zwei Sequenzen zu optimieren, und somit einen aussagekräftigeren Vergleich der beiden Sequenzen erreichen.

Die prozentuale Identität zwischen Sequenzen kann unter Verwendung von ein oder mehreren Computeralgorithmen oder im Stand der Technik bekannten oder hierin beschriebenen Programmen bestimmt werden. Erfindungsgemäß wird zur Bestimmung der Identität das durch National Center for Biotechnology Information (NCBI) bereitgestellte Basic Local Alignment Search Tool (BLAST) (Altschul et al., 1990) verwendet. Die BLAST-Software-Reihe enthält verschiedene Programme, einschließlich ein als "BLAST 2 Sequences" genanntes Werkzeug, das für den direkten paarweisen Vergleich von zwei Nukleotid- bzw. Aminosäuresequenzen verwendet wird. "BLAST 2 Sequences" kann auch über die NCBI World Wide Web-Seite im Internet interaktiv abgerufen und verwendet werden. Das blastn-Programm (für Nukleotidsequenzen) verwendet als Vorgaben eine Wortlänge (W) von 11, eine Erwartung (E) von 10, M = 5, N =-4 und einen Vergleich beider Stränge. Für Aminosäuresequenzen verwendet das blastp-Programm als Vorgaben eine Wortlänge von 3 und eine Erwartung (E) von 10 und die BLOSUM62-Scoring-Matrix (Henikoff & Henikoff, 1989), Alignments (B) von 50, Erwartung (E) von 10, M = 5, N = - 4.

Alternativ umfasst die Oxidoreduktase zur Reduktion von D-Psicose zu Allitol vorzugsweise eine Aminosäuresequenz, die durch eine Nukleinsäure kodiert wird, die unter stringenten Bedingungen an ein Nukleinsäuremolekül mit der Nukleinsäuresequenz SEQ ID Nr. 1 bindet. Wie hierin verwendet, beziehen sich die stringenten Bedingungen auf Bedingungen, unter denen sogenannte spezifische Hybride, jedoch keine unspezifischen Hybride gebildet werden. Beispielsweise umfassen die stringenten Bedingungen eine Hybridisierung in 6xSSC (Natriumchlorid/Natriumcitrat) bei 45 °C und dann Waschen mit 0,2 bis 1xSSC, 0,1% SDS bei 50 bis 65 °C; oder derartige Bedingungen können eine Hybridisierung in 1xSSC bei 65 bis 70 °C und dann Waschen mit 0,3xSSC bei 65 bis 70°C umfassen. Die Hybridisierung kann durch herkömmlich bekannte Verfahren, wie etwa jene, die von J. Sambrook et al. in Molecular Cloning, A Laboratory Manual, 2nd Ed., Cold Spring Harbor Laboratory (1989), beschrieben sind, durchgeführt werden.

Ein Aspekt der vorliegenden Erfindung betrifft die Verwendung einer Oxidoreduktase zur Reduktion von D-Psicose zu Allitol, wobei die Oxidoreduktase eine Aminosäuresequenz umfasst oder aus dieser besteht, welche ausgewählt ist aus der Gruppe bestehend aus:
i) einer Aminosäuresequenz, die eine Identität zu SEQ ID Nr. 2 von mindestens 80% aufweist,
ii) einer Aminosäuresequenz, die durch eine Nukleinsäure kodiert wird, die eine Identität zu SEQ ID Nr. 1 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 1 bindet.

Die zur Kofaktor-Regeneration eingesetzte Alkoholdehydrogenase (ADH) kann aus einer der Gruppen EC 1.1.1.1 (NAD-abhängige ADH) und EC 1.1.1.2 (NADP-abhängige ADH) stammen.

Die NAD(P)-abhängige Alkoholdehydrogenase zur Kofaktor-Regeneration umfasst oder besteht vorzugsweise aus einer Aminosäuresequenz, welche ausgewählt ist aus der Gruppe bestehend aus:
i) einer Aminosäuresequenz, die eine Identität zu SEQ ID Nr. 4 von mindestens 80% aufweist,
ii) einer Aminosäuresequenz, die durch eine Nukleinsäure kodiert wird, die eine Identität zu SEQ ID Nr. 3 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 3 bindet.

Besonders geeignet zur Kofaktor-Regeneration im Allgemeinen ist eine Alkoholdehydrogenase, deren Aminosäuresequenz mindestens 80% ident zu SEQ ID Nr. 4 ist bzw. welche durch eine Nukleinsäure kodiert wird, die eine Identität zu SEQ ID Nr. 3 von mindestens 80% aufweist bzw. unter stringenten Bedingungen an ein Nukleinsäuremolekül mit der Nukleinsäuresequenz SEQ ID Nr. 3 bindet.
SEQ ID Nr. 3: 
ATGAAAGCTGCAGTTGTGGAACAATTTAAAAAGCCGTTACAAGTGAAAGAAGTGGAAAAACCTAAGAT
CTCATACGGGGAAGTATTAGTGCGCATCAAAGCGTGTGGGGTATGCCATACAGACTTGCATGCCGCAC
ATGGCGACTGGCCTGTAAAGCCTAAACTGCCTCTCATTCCTGGCCATGAAGGCGTCGGTGTAATTGAAG
AAGTAGGTCCTGGGGTAACACATTTAAAAGTTGGAGATCGCGTAGGTATCCCTTGGCTTTATTCGGCGT
GCGGTCATTGTGACTATTGCTTAAGCGGACAAGAAACATTATGCGAACGTCAACAAAACGCTGGCTATT
CCGTCGATGGTGGTTATGCTGAATATTGCCGTGCTGCAGCCGATTATGTCGTAAAAATTCCTGATAACTT
ATCGTTTGAAGAAGCCGCTCCAATCTTTTGCGCTGGTGTAACAACATATAAAGCGCTCAAAGTAACAGG
CGCAAAACCAGGTGAATGGGTAGCCATTTACGGTATCGGCGGGCTTGGACATGTCGCAGTCCAATACG
CAAAGGCGATGGGGTTAAACGTCGTTGCTGTCGATTTAGGTGATGAAAAACTTGAGCTTGCTAAACAA
CTTGGTGCAGATCTTGTCGTCAATCCGAAACATGATGATGCAGCACAATGGATAAAAGAAAAAGTGGG
CGGTGTGCATGCGACTGTCGTCACAGCTGTTTCAAAAGCCGCGTTCGAATCAGCCTACAAATCCATTCG
 
TCGCGGTGGTGCTTGCGTACTCGTCGGATTACCGCCGGAAGAAATACCTATTCCAATTTTCGATACAGT
ATTAAATGGAGTAAAAATTATTGGTTCTATCGTTGGTACGCGCAAAGACTTACAAGAGGCACTTCAATT
TGCAGCAGAAGGAAAAGTAAAAACAATTGTCGAAGTGCAACCGCTTGAAAACATTAACGACGTATTCG
ATCGTATGTTAAAAGGGCAAATTAACGGCCGCGTCGTGTTAAAAGTAGATTAA
SEQ ID Nr. 4:

MKAAVVEQFKKPLQVKEVEKPKISYGEVLVRIKACGVCHTDLHAAHGDWPVKPKLPLIPGHEGVGVIEEV
GPGVTHLKVGDRVGIPWLYSACGHCDYCLSGQETLCERQQNAGYSVDGGYAEYCRAAADYVVKIPDNLS
FEEAAPIFCAGVTTYKALKVTGAKPGEWVAIYGIGGLGHVAVQYAKAMGLNVVAVDLGDEKLELAKQLG
ADLVVNPKHDDAAQWIKEKVGGVHATVVTAVSKAAFESAYKSIRRGGACVLVGLPPEEIPIPIFDTVLNGV
KIIGSIVGTRKDLQEALQFAAEGKVKTIVEVQPLENINDVFDRMLKGQINGRVVLKVD

Die hier angeführte Alkoholdehydrogenase zur Kofaktor-Regeneration umfasst vorzugsweise eine Aminosäuresequenz, die eine Identität zu SEQ ID Nr. 4 von mindestens 80%, noch mehr bevorzugt von 85%, noch mehr bevorzugt von 90%, noch mehr bevorzugt von 95%, noch mehr bevorzugt von 98%, noch mehr bevorzugt von 99%, insbesondere von 100%, aufweist. Besonders bevorzugt umfasst die erfindungsgemäße Alkoholdehydrogenase zur Kofaktor-Regeneration die Aminosäuresequenz SEQ ID Nr. 4 oder besteht aus dieser.

Alternativ umfasst die Alkoholdehydrogenase zur Kofaktor-Regeneration vorzugsweise eine Aminosäuresequenz, die durch eine Nukleinsäure kodiert wird, die eine Identität zu SEQ ID Nr. 3 von mindestens 80%, noch mehr bevorzugt von 85%, noch mehr bevorzugt von 90%, noch mehr bevorzugt von 95%, noch mehr bevorzugt von 98%, noch mehr bevorzugt von 99%, insbesondere von 100%, aufweist oder besteht aus dieser. Besonders bevorzugt umfasst die Nukleinsäure, welche die erfindungsgemäße Alkoholdehydrogenase zur Kofaktor-Regeneration kodiert, die Nukleinsäuresequenz SEQ ID Nr. 3 oder besteht aus dieser.

Ein Aspekt der vorliegenden Erfindung betrifft die Verwendung einer Alkoholdehydrogenase zur Kofaktor-Regeneration, wobei die Alkoholdehydrogenase eine Aminosäuresequenz umfasst oder aus dieser besteht, welche ausgewählt ist aus der Gruppe bestehend aus:
i) einer Aminosäuresequenz, die eine Identität zu SEQ ID Nr. 4 von mindestens 80% aufweist,
ii) einer Aminosäuresequenz, die durch eine Nukleinsäure kodiert wird, die eine Identität zu SEQ ID Nr. 3 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 3 bindet.

Die zur Kofaktor-Regeneration eingesetzte Glucose-Dehydrogenase (GDH) kann aus einer der Gruppen EC 1.1.1.47 (Glucose-1-dehydrogenase), EC 1.1.1.118 (Glucose-1-dehydrogenase (NAD⁺)), EC 1.1.1.119 (Glucose-1-dehydrogenase (NADP⁺)) oder EC 1.1.1.360 (Glucose/Galactose-1-dehydrogenase) stammen.

Die NAD(P)-abhängige Glucose-Dehydrogenase zur Kofaktor-Regeneration umfasst oder besteht vorzugsweise aus einer Aminosäuresequenz, welche ausgewählt ist aus der Gruppe bestehend aus:
i) einer Aminosäuresequenz, die eine Identität zu SEQ ID Nr. 6 von mindestens 80% aufweist,
ii) einer Aminosäuresequenz, die durch eine Nukleinsäure kodiert wird, die eine Identität zu SEQ ID Nr. 5 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 5 bindet.

Besonders geeignet zur Kofaktor-Regeneration im Allgemeinen ist eine Glucose-Dehydrogenase, deren Aminosäuresequenz mindestens 80% ident zu SEQ ID Nr. 6 ist bzw. welche durch eine Nukleinsäure kodiert wird, die eine Identität zu SEQ ID Nr. 5 von mindestens 80% aufweist bzw. unter stringenten Bedingungen an ein Nukleinsäuremolekül mit der Nukleinsäuresequenz SEQ ID Nr. 5 bindet.
SEQ ID Nr. 5: 
ATGTATACAGATTTAAAAGATAAAGTAGTTGTAATTACAGGTGGATCAACAGGTTTAGGACGCGCAA
TGGCTGTTCGTTTCGGTCAAGAAGAAGCAAAAGTTGTTATTAACTATTACAACAATGAAGAAGAAGCT
TTAGATGCGAAAAAAGAAGTAGAAGAAGCAGGCGGACAAGCAATCATCGTTCAAGGCGACGTAACA
AAAGAAGAAGATGTTGTAAACCTTGTTCAAACAGCTATTAAAGAATTCGGTACATTAGACGTTATGAT
TAATAACGCTGGTGTTGAAAACCCAGTTCCTTCTCATGAGTTATCTTTAGACAACTGGAATAAAGTTAT
TGATACAAACTTAACAGGTGCATTCTTAGGAAGCCGTGAAGCAATCAAATATTTTGTTGAAAACGACA
TTAAAGGAAACGTTATTAACATGTCTAGTGTTCATGAAATGATTCCTTGGCCATTATTTGTTCATTACG
CAGCAAGTAAAGGCGGTATGAAACTAATGACGGAAACATTGGCTCTTGAATATGCGCCAAAAGGTAT
CCGCGTAAATAACATTGGACCAGGTGCGATGAACACACCAATTAACGCAGAGAAATTTGCAGATCCT
GTACAACGTGCAGACGTAGAAAGCATGATTCCAATGGGTTACATCGGTAAACCAGAAGAAGTAGCA
 
GCAGTTGCAGCATTCTTAGCATCATCACAAGCAAGCTATGTAACAGGTATTACATTATTTGCTGATGG
TGGTATGACGAAATACCCTTCTTTCCAAGCAGGAAGAGGCTAA
SEQ ID Nr. 6:

MYTDLKDKVVVITGGSTGLGRAMAVRFGQEEAKVVINYYNNEEEALDAKKEVEEAGGQAIIVQGDVTKEED
VVNLVQTAIKEFGTLDVMINNAGVENPVPSHELSLDNWNKVIDTNLTGAFLGSREAIKYFVENDIKGNVIN
MSSVHEMIPWPLFVHYAASKGGMKLMTETLALEYAPKGIRVNNIGPGAMNTPINAEKFADPVQRADVES
MIPMGYIGKPEEVAAVAAFLASSQASYVTGITLFADGGMTKYPSFQAGRG

Die hier angeführte Glucose-Dehydrogenase zur Kofaktor-Regeneration umfasst vorzugsweise eine Aminosäuresequenz, die eine Identität zu SEQ ID Nr. 6 von mindestens 80%, noch mehr bevorzugt von 85%, noch mehr bevorzugt von 90%, noch mehr bevorzugt von 95%, noch mehr bevorzugt von 98%, noch mehr bevorzugt von 99%, insbesondere von 100%, aufweist. Besonders bevorzugt umfasst die erfindungsgemäße Glucose-Dehydrogenase zur Kofaktor-Regeneration die Aminosäuresequenz SEQ ID Nr. 6 oder besteht aus dieser.

Alternativ umfasst die Glucose-Dehydrogenase zur Kofaktor-Regeneration vorzugsweise eine Aminosäuresequenz, die durch eine Nukleinsäure kodiert wird, die eine Identität zu SEQ ID Nr. 5 von mindestens 80%, noch mehr bevorzugt von 85%, noch mehr bevorzugt von 90%, noch mehr bevorzugt von 95%, noch mehr bevorzugt von 98%, noch mehr bevorzugt von 99%, insbesondere von 100%, aufweist. Besonders bevorzugt umfasst die Nukleinsäure, welche die erfindungsgemäße Glucose-Dehydrogenase zur Kofaktor-Regeneration kodiert, die Nukleinsäuresequenz SEQ ID Nr. 5 oder besteht aus dieser.

Ein Aspekt der vorliegenden Erfindung betrifft die Verwendung einer Glucose-Dehydrogenase zur Kofaktor-Regeneration, wobei die Glucose-Dehydrogenase eine Aminosäuresequenz umfasst oder aus dieser besteht, welche ausgewählt ist aus der Gruppe bestehend aus:
i) einer Aminosäuresequenz, die eine Identität zu SEQ ID Nr. 6 von mindestens 80% aufweist,
ii) einer Aminosäuresequenz, die durch eine Nukleinsäure kodiert wird, die eine Identität zu SEQ ID Nr. 5 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 5 bindet.

Die hier vorgestellte enzymatische Strategie in Kombination mit der Kofaktor-Regeneration ermöglicht einen biokatalytischen, umweltfreundlichen und hocheffizienten Produktionsprozess zur Herstellung von Allitol.

### Materialien

D-Psicose wurde von TCI und Hunan Garden Naturals Inc. (China), Allitol wurde von TCI, D-Fructose, Lysozym sowie Methanol wurden von PanReac AppliChem (ITW Reagents), D-Glucose, Natriumgluconat, IPTG (Isopropyl-β-D-thiogalactopyranosid) wurden von Sigma-Aldrich, Kaliumdihydrogenphosphat, di-Kaliumhydrogenphosphat, NAD⁺, NADH-Dinatriumsalz sowie Natriumdodecylsulfat (SDS) wurden von Carl Roth und Triethanolamin (TEA) wurde von Chem-Lab NV bezogen.

### Produktion der Enzyme & Herstellung der Lysate

### Allgemeines zur Expression von rekombinanten Enzymen in E. coli

Für die rekombinante Enzymproduktion in einem *Escherichia coli*-Stamm wurde zunächst das zu exprimierende Gen in einer PCR unter der Verwendung der genomischen DNA oder deren synthetisch an die Codon-Verwendung von *E. coli* angepasstes Äquivalent als Matrize zusammen mit spezifischen Oligonukleotiden, die zusätzlich Erkennungssequenzen für Restriktionsendonukleasen tragen, amplifiziert und aus dem Reaktionsgemisch isoliert. Nach dem Nukleinsäure-Verdau mit den Restriktionsenzymen Sphl und Hindlll wurde das für das Target-Enzym kodierende Genfragment in das mit Sphl und Hindlll geschnittene Rückgrat des Expressionsvektors pQE70-Kan ligiert. Das Ligationsprodukt wurde in chemisch kompetente *E*. *coli*-Zellen Top10F transformiert und die entstandenen Kolonien wurden für die Plasmid-Isolierung und Restriktionsanalyse benutzt.

Das Ergebnis des Klonierungs-Schritts wurde mittels Restriktionsenzym-Verdau und DNA-Sequenzierung überprüft. Das resultierende Konstrukt trägt das Target-Gen unter dem IPTGinduzierbaren T5-Promotor.

Für die Überexpression des Enzymes in *E. coli* wurde das resultierende Expressionsplasmid in die kompetenten Expressionszellen RB791 transformiert. Nach 24 h Inkubation bei 37 °C wurden entstandene Kolonien für die Expressionstests in LB-Medium angeimpft.

Am nächsten Tag wurden damit Expressionskulturen mit einer optische Dichte OD₅₅₀ von 0,02 angeimpft und bei 37 °C geschüttelt, bis eine OD₅₅₀ von 0,3 erreicht wurde. Anschließend wurde die Temperatur auf 25 °C gesenkt und die Kulturen wurden beim Erreichen einer OD₅₅₀ von 0,5 mit 0,1 mM IPTG induziert. Nach 22 h wurden die Kulturen geerntet (vom Medium mittels Zentrifugation in Form eines Zellpellets abgetrennt) und auf die Expression des rekombinanten Enzymes mit Hilfe von SDS-Gelelektrophorese und einer Aktivitätsbestimmung (Verwendung in Use-Test oder optisch-enzymatischer Assay) analysiert.

### Herstellung von Zell-Lysaten mittels Sonifier-Aufschlusses

Zur Herstellung einer Zell-Suspension wurde das nach obigen Verfahren hergestellte Zellpellet in einem geeigneten Gefäß eingewogen und mit Puffer und Lysozym (finale Konzentration 0,5 mg/ml) versetzt (z.B. Triethanolamin (TEA) - HCl) und unter Rührung gelöst. Der Massenanteil an Biomasse beträgt üblicherweise 20%, der Rest entfällt auf den Puffer.

Zum Zell-Aufschluss wurde ein Branson Sonifier 450 verwendet. Die Suspension wurde dreimal mit je 15 Ultraschall-Stößen (Einstellungen am Gerät: Timer = 15; Duty Cycle = 50; Output Control = 3 - 5) behandelt.

Das erhaltene Homogenat wurde 10 min lang bei 4 °C und 16000 rpm zentrifugiert (Eppendorf Zentrifuge 5417R), um die unlöslichen Zellfragmente abzutrennen und das Lysat zu erhalten.

**Tabelle 1. Enzymklassen und Spenderorganismen für die in den Beispielen verwendeten Enzyme.**

| **Enzymtyp (EC-Klasse)** | **katalysierte Reaktion** | **Spenderorganismus** | **Literatur** |
|---|---|---|---|
| D-Psicose-3-Epimerase (EC 5.1.3.30) | D-Fructose → D-Psicose | *Clostridium cellulolyticum* H10 | (Mu et al., 2011; Chan et al., 2012) |
| Short-Chain-Dehydrogenase/Reduktase | D-Psicose → Allitol | *Gluconobacter frateurii* (DSM 7146) | (NCBI Protein Database: WP_063903495.1); SEQ ID Nr. 2 |
| Ribitol-Dehydrogenase (EC 1.1.1.56) | D-Psicose → Allitol | *Klebsiella pneumoniae* | (Takeshita et al., 2000; NCBI Protein Database: WP_265716617.1) |
| Alkoholdehydrogenase (ADH) (EC 1.1.1.1) | 2-Propanol → Aceton | *(Geo-)Bacillus stearothermophilus* NCA1503 | (Sakoda & Imanaka, 1992; NCBI Protein Database: WP_033015595.1); SEQ ID Nr. 4 |
| Glucose-Dehydrogenase (GDH) | D-Glucose → D-Gluconat (via D-Gluconolacton) | *Priestia megaterium* | (NCBI Protein Database: MDQ0804260.1); SEQ ID Nr. 6 |

### Analytische Methoden

### High Performance Liquid Chromatography

Zur Quantifizierung von D-Psicose, D-Fructose, D-Glucose sowie Allitol mittels HPLC (High Performance Liquid Chromatography) wurde ein Agilent HPLC 1260 Infinity II Series System verwendet. Die Detektion erfolgte mittels eines Brechungsindex-Detektors (RI-Detektion). Zur Messung wurde eine Phenomenex Rezex RPM-Monosaccharide Pb+2 (8%) Säule mit entsprechender Vorsäule verwendet und mit Reinstwasser isokratisch eluiert.

### High Performance Anion Exchange Chromatography

Zur Quantifizierung von D-Gluconsäure/D-Gluconat mittels HPAEC (High Performance Anion Exchange Chromatography) wurde ein Dionex ICS6000 System mit AS-AP Autosampler verwendet. Die Messung erfolgte mittels Leitfähigkeitsdetektion (CD) gekoppelt an einen Dionex AERS 500 elektrolytisch regenerierten Suppressor im externen Wassermodus. Zur Trennung der Analyten wurde eine Dionex IonPac AS11-HC-4µm Säule mit entsprechender Vorsäule sowie einem NaOH-Gradienten verwendet. Das Laufmittel wurde zusätzlich mit einer Dionex ATC Anion Trap Column vorbehandelt.

### Bestimmung von Enzym-Aktivitäten (optisch-enzymatischer Assay)

Enzym-Aktivitäten in den Lysaten wurden mit einem Shimadzu UV-1900 Spektrophotometer bestimmt. Dazu wurde die Bildung oder der Verbrauch von NAD(P)H bei einer Wellenlänge von 340 nm über die Änderung der Absorption verfolgt. Die Messungen wurden mit 0,2 mM Kofaktor (NAD(P)⁺ oder NAD(P)H) durchgeführt. Dazu wurden 20 µl einer 10 mM Stock-Lösung des Kofaktors in einer Küvette (Greiner bio-one Semi-Micro-Küvette aus Polystyrol) vorgelegt und der gewünschte pH-Wert wurde mit 100 mM TEA-HCl-Puffer eingestellt (870 µl). 10 µl Lysat (verdünnt oder unverdünnt) und 100 µl Substrat-Lösung wurden zur Küvette zugesetzt und die Messung unmittelbar danach gestartet. Die Messungen wurden standardmäßig bei 25 °C durchgeführt. Über den Extinktionskoeffizienten von NADH/NADPH bei 340 nm (*ε* = 6220 L mol⁻¹ cm⁻¹) kann die Enzym-Aktivität des Lysats in U/ml (bezogen auf das Volumen des Lysats) oder U/g (bezogen auf die zur Herstellung eingesetzte Biomasse) bestimmt werden. 1 U steht dabei für 1 µmol Substratumsatz pro Minute (1 U = 1 µmol/min = 1,67·10⁻⁸ kat).

Mit den nachfolgenden Beispielen werden bevorzugte Varianten des erfindungsgemäßen Verfahrens noch näher beschrieben. Die in diesen Beispielen eingesetzten Lysate wurden nach den oben beschriebenen Verfahren hergestellt.

### Beispiel 1

### Umsetzung von D-Fructose zu Allitol - Kofaktor-Regeneration durch ADH und 2-Propanol

In einem offenen 2 ml Glas-Vial wurden folgende Komponenten vermischt: 189,3 µl deionisiertes Wasser, 50 µl eines 500 mM TEA-HCl-Puffers (pH 8), 100 µl einer D-Fructose-Lösung (500 g/l) sowie 35 µl D-Psicose-3-Epimerase-Lysat. Anschließend wurden 50 µl Short-Chain-Dehydrogenase/Reduktase-Lysat, 12 U Alkoholdehydrogenase-Lysat, 10 µl einer 10 mM NAD⁺-Lösung sowie 50 µl 2-Propanol zum Ansatz hinzugefügt. Der Ansatz wurde unter kontinuierlichem Schütteln (Eppendorf-Thermomixer, 35 °C, 1200 rpm) für insgesamt 56 h inkubiert.

Nach 24 und 48 h wurden jeweils 50 µl einer 2-Propanol/Wasser-Mischung (2/3 v/v) zum Ansatz hinzugefügt.

Zur Analyse wurden 100 µl des Ansatzes mit 200 µl Methanol versetzt und im Eppendorf Thermomixer bei 60 °C und 1200 rpm für 15 min inkubiert. Die Probe wurde in einer Zentrifuge kurz zentrifugiert, mit 700 µl deionisiertem Wasser versetzt, gevortext und anschließend für 5 min bei max. g zentrifugiert. 200 µl des Überstands wurden in ein HPLC-Vial mit Insert überführt und mittels HPLC (RI-Detektion) vermessen.

Auf diese Weise wurden 94,2% der D-Fructose (100 g/l) zu Allitol (gefundene Konzentration: 83,0 g/l) umgesetzt.

Zur Kristallisation wurden 100 ml derselben Mischung verwendet, auf pH 4 angesäuert und bei 70 °C für 30 min gerührt. Die heiße Reaktionsmischung wurde durch eine Glasfritte (P4, 10-16 µm) mittels Anlegens eines Vakuums filtriert. Das Filtrat wurde zuerst auf Raumtemperatur abgekühlt und danach über Nacht im Kühlschrank bei 4 °C gelagert. Der farblose Niederschlag wurde abfiltriert (Glasfritte P4, 10-16 µm) und für 24 h im Vakuumtrockenschrank bei 50 °C getrocknet.

Das Filtrat wurde mit Aceton versetzt und über Nacht im Kühlschrank bei 4 °C gelagert. Der Niederschlag wurde abfiltriert (Glasfritte P4, 10-16 µm) und mit eiskaltem Aceton gewaschen. Das Produkt wurde für 24 h im Vakuumtrockenschrank bei 50 °C getrocknet. Eine HPLC-Analyse der Produktfraktionen ergab, dass es sich bei dem Produkt um Allitol handelte, welches in hoher Reinheit (≥ 99%) gewonnen werden konnte.

Das Beispiel zeigt, dass mit dem hier beschriebenen Enzymsystem bestehend aus DPE, Short-Chain-Dehydrogenase/Reduktase sowie ADH die effiziente Umsetzung von D-Fructose (100 g/l) zu Allitol in der 10-fachen Substratkonzentration im Vergleich zu Takeshita et al. (2000) möglich ist.

### Beispiel 2

### Umsetzung von D-Fructose zu Allitol - Kofaktor-Regeneration durch GDH und D-Glucose

Die Reaktion wurde in einem Multifors Tisch-Bioreaktor (Infors AG) durchgeführt. Als Gefäß wurde ein Glasreaktor (Volumen 1 l) mit aufgesetztem Rührwerk und pH-Elektrode verwendet. Die pH-Kontrolle erfolgte durch die Zudosierung von 5M NaOH oder 1M H₂SO₄.

Zu Beginn wurden 52,5 g D-Fructose, 52,5 g D-Glucose, 172,5 ml deionisiertes Wasser, 26,5 ml eines 500 mM KPP-Puffers (pH 7,5) im Reaktor vorgelegt und unter Rühren auf 35 °C gebracht.

Zum Start der Reaktion wurden 17,5 ml D-Psicose-3-Epimerase-Lysat zugesetzt. Danach wurden 24,5 ml Short-Chain-Dehydrogenase/Reduktase-Lysat, 2,2 kU Glucose-Dehydrogenase-Lysat sowie 3,5 ml einer 10 mM NAD⁺-Lösung eingebracht.

Zur Analyse wurden 50 µl des Ansatzes mit 200 µl Methanol versetzt und im Eppendorf Thermomixer bei 60 °C und 1200 rpm für 15 min inkubiert. Die Probe wurde in einer Zentrifuge kurz zentrifugiert, mit 750 µl deionisiertem Wasser versetzt, gevortext und anschließend für 5 min bei max. g zentrifugiert. 200 µl des Überstands wurden in ein HPLC-Vial mit Insert überführt und mittels HPLC (RI-Detektion) vermessen. Für die HPAEC-Messungen (Leitfähigkeitsdetektion) wurde der klare Überstand 1:250 verdünnt.

Auf diese Weise wurden in 30 h 90,6% der D-Fructose (150 g/l) zu Allitol (gefundene Konzentration: 119 g/l) und die D-Glucose (150 g/l) vollständig zu D-Gluconat (gefundene Konzentration: 167 g/l) umgesetzt.

Der Reaktorinhalt wurde 1 h bei 70 °C gerührt. Die heiße Reaktionsmischung wurde durch eine Glasfritte (P4, 10-16 µm) mittels Anlegens eines Vakuums filtriert. Das Filtrat wurde mit Aceton versetzt (finaler Volumenanteil 50 %) und über Nacht im Kühlschrank bei 4 °C gelagert. Der Niederschlag wurde abfiltriert (Glasfritte P4, 10-16 µm) und mit eiskaltem Aceton gewaschen. Das Produkt wurde für 24 h im Vakuumtrockenschrank bei 50 °C getrocknet. Eine HPLC und HPAEC-Analyse des Produkts ergab, dass Allitol in hoher Reinheit (≥ 97%) aus der D-Gluconat-haltigen Lösung gewonnen werden konnte.

Das Beispiel zeigt, dass mit dem hier beschriebenen Enzymsystem bestehend aus DPE, Short-Chain-Dehydrogenase/Reduktase sowie GDH die effiziente Umsetzung von D-Fructose (150 g/l) zu Allitol in der 15-fachen Substratkonzentration im Vergleich zu Takeshita et al. (2000) möglich ist.

### Literatur

Hough, L., & Stacey, B. E. (1963). The occurrence of D-ribohexulose in Itea ilicifolia, Itea virginica, and Itea yunnanensis. Phytochemistry, 2(4), 315-320. https://doi.org/10.1016/S0031-9422(00)84854-2
Izumori, K. (2006). Izumoring: a strategy for bioproduction of all hexoses. Journal of Biotechnology, 124(4), 717-722. https://doi.org/10.1016/j.jbiotec.2006.04.016
Hassanin, H. A. M., Mu, W., Koko, M. Y. F., Zhang, T., Masamba, K., & Jiang, B. (2017). Allitol: production, properties and applications. International Journal of Food Science and Technology, 52(1), 91-97. https://doi.org/10.1111/ijfs.13290
Gullapalli, P., Takata, G., Poonperm, W., Rao, D., Morimoto, K., Akimitsu, K., Tajima, S., & Izumori, K. (2007). Bioproduction of D-psicose from allitol with Enterobacter aerogenes IK7: a new frontier in rare ketose production. Bioscience, Biotechnology, and Biochemistry, 71(12), 3048-3054. https://doi.org/10.1271/bbb.70450
Poonperm, W., Takata, G., Ando, Y., Sahachaisaree, V., Lumyong, P., Lumyong, S., & Izumori, K. (2007). Efficient conversion of allitol to D-psicose by Bacillus pallidus Y25. Journal of Bioscience and Bioengineering, 103(3), 282-285. https://doi.org/10.1263/jbb.103.282
Takeshita, K., Shimonishi, T., & Izumori, K. (1996). Production of L-psicose from allitol by Gluconobacter frateurii IFO 3254. Journal of Fermentation and Bioengineering, 81(3), 212-215. https://doi.org/10.1016/0922-338X(96)82210-0
Izumori, K., Khan, A. R., Okaya, H., & Tsumura, T. (1993). A New Enzyme, D-Ketohexose 3-Epimerase, from Pseudomonas sp. ST-24. Bioscience, Biotechnology, and Biochemistry, 57(6), 1037-1039. https://doi.org/10.1271/bbb.57.1037
Zhang, W., Yu, S., Zhang, T., Jiang, B., & Mu, W. (2016). Recent advances in D-allulose: Physiological functionalities, applications, and biological production. Trends in Food Science and Technology, 54, 127-137. https://doi.org/10.1016/j.tifs.2016.06.004
Jiang, S., Xiao, W., Zhu, X., Yang, P., Zheng, Z., Lu, S., Jiang, S., Zhang, G., & Liu, J. (2020). Review on D-Allulose: In vivo Metabolism, Catalytic Mechanism, Engineering Strain Construction, Bio-Production Technology. Frontiers in Bioengineering and Biotechnology, 8, 26. https://doi.org/10.3389/fbioe.2020.00026
Muniruzzaman, S., Tokunaga, H., & Izumori, K. (1995). Conversion of D-psicose to allitol by Enterobacter agglomerans strain 221e. Journal of Fermentation and Bioengineering, 79(4), 323-327. https://doi.org/10.1016/0922-338X(95)93989-W
Han, W., Zhu, Y., Men, Y., Yang, J., Liu, C., & Sun, Y. (2014). Production of allitol from D-psicose by a novel isolated strain of Klebsiella oxytoca G4A4. Journal of Basic Microbiology, 54(10), 1073-1079. https://doi.org/10.1002/jobm.201300647
Zhu, Y., Li, H., Liu, P., Yang, J., Zhang, X., & Sun, Y. (2015). Construction of allitol synthesis pathway by multi-enzyme coexpression in Escherichia coli and its application in allitol production. Journal of Industrial Microbiology & Biotechnology, 42(5), 661-669. https://doi.org/10.1007/s10295-014-1578-1
Wen, X., Lin, H., Ren, Y., Li, C., Zhang, C., Lin, J., & Lin, J. (2022). Allitol bioproduction by recombinant Escherichia coli with NADH regeneration system co-expressing ribitol dehydrogenase (RDH) and formate dehydrogenase (FDH) in individual or in fusion. Electronic Journal of Biotechnology, 55, 91-98. https://doi.org/10.1016/j.ejbt.2021.11.007
Wang, L., Chen, K., Zheng, P., Huo, X., Liao, F., Zhu, L., Hu, M., & Tao, Y. (2023). Enhanced production of D-psicose from D-fructose by a redox-driven multi-enzyme cascade system. Enzyme and Microbial Technology, 163, 110172. https://doi.org/10.1016/j.enzmictec.2022.110172
Neuhauser, W., Steininger, M., Haltrich, D., Kulbe, K. D., & Nidetzky, B. (1998). A pH-Controlled Fed-Batch Process Can Overcome Inhibition by Formate in NADH-Dependent Enzymatic Reductions Using Formate Dehydrogenase-Catalyzed Coenzyme Regeneration. Biotechnology and Bioengineering, 60(3), 277-282. https://doi.org/10.1002/(SICI)1097-0290(19981105)60:3<277::AID-BIT2>3.0.CO;2-E
Kratzer, R., Woodley, J. M., & Nidetzky, B. (2015). Rules for biocatalyst and reaction engineering to implement effective, NAD(P)H-dependent, whole cell bioreductions. Biotechnology Advances, 33(8), 1641-1652. https://doi.org/10.1016/j.biotechadv.2015.08.006
Boldt, A., & Ansorge-Schumacher, M. B. (2020). Formate Dehydrogenase from Rhodococcus jostii (RjFDH) - A High-Performance Tool for NADH Regeneration. Advanced Synthesis und Catalysis, 362(19), 4109-4118. https://doi.org/10.1002/adsc.202000536
Tishkov, V. I., & Popov, V. O. (2004). Catalytic Mechanism and Application of Formate Dehydrogenase. Biochemistry (Moscow), 69(11), 1252-1267. https://doi.org/10.1007/s10541-005-0071-x
Zhao, J., Guo, Y., Li, Q., Chen, J., Niu, D., & Liu, J. (2022). Reconstruction of a Cofactor Self-Sufficient Whole-Cell Biocatalyst System for Efficient Biosynthesis of Allitol from D-Glucose. Journal of Agricultural and Food Chemistry, 70(12), 3775-3784. https://doi.org/10.1021/acs.jafc.2c00440
Feng, T., Wang, Z., Li, H., Li, Q., Guo, Y., Zhao, J., & Liu, J. (2023). Whole-cell biotransformation for simultaneous synthesis of allitol and D-gluconic acid in recombinant Escherichia coli. Journal of Bioscience and Bioengineering, 135(6), 433-439. https://doi.org/10.1016/j.jbiosc.2023.03.004
Hassanin, H. A. M., Letsididi, R., Koko, M. Y. F., Mu, W., Elferga, A., & Jiang, B. (2016). Synthesis of allitol from D-psicose using ribitol dehydrogenase and formate dehydrogenase. Tropical Journal of Pharmaceutical Research, 15(12), 2701-2708. https://doi.org/10.4314/tjpr.v15i12.23
Xu, J., Zhou, H., Yu, H., Deng, T., Wang, Z., Zhang, H., Wu, J., & Yang, L. (2021). Computational design of highly stable and soluble alcohol dehydrogenase for NADPH regeneration. Bioresources and Bioprocessing, 8, 12. https://doi.org/10.1186/s40643-021-00362-w
Takeshita, K., Ishida, Y., Takada, G., & Izumori, K. (2000). Direct Production of Allitol from D-Fructose by a Coupling Reaction Using D-Tagatose 3-Epimerase, Ribitol Dehydrogenase and Formate Dehydrogenase. Journal of Bioscience and Bioengineering, 90(5), 545-548. https://doi.org/10.1016/51389-1723(01)80038-4
Al-Rabiah, A. A., Boz, I., Akhmedov, V. M., Mostafa, M. M. M., & Bagabas, A. A. (2022). Highly Selective Gas-Phase Catalytic Hydrogenation of Acetone to Isopropyl Alcohol. Catalysts, 12(10), 1251. https://doi.org/10.3390/catal12101251
Kornecki, J. F., Carballares, D., Tardioli, P. W., Rodrigues, R. C., Berenguer-Murcia, A., Alcäntara, A. R., & Fernandez-Lafuente, R. (2020). Enzyme production of D-gluconic acid and glucose oxidase: successful tales of cascade reactions. Catalysis Science & Technology, 10(17), 5740-5771. https://doi.org/10.1039/D0CY00819B
Lin, B., & Tao, Y. (2017). Whole-cell biocatalysts by design. Microbial Cell Factories, 16, 106. https://doi.org/10.1186/s12934-017-0724-7
Altschul, S. F., Gish, W., Miller, W., Myers, E. W., & Lipman, D. J. (1990). Basic local alignment search tool. Journal of Molecular Biology, 215(3), 403-410. https://doi.org/10.1016/S0022-2836(05)80360-2
Henikoff, S., & Henikoff, J. G. (1992). Amino acid substitution matrices from protein blocks. Proceedings of the National Academy of Sciences of the United States of America, 89(22), 10915-10919. https://doi.org/10.1073/pnas.89.22.10915
Sambrook, J., Fritsch, E. R., & Maniatis, T. (1989). Molecular Cloning: A Laboratory Manual (2nd ed.). Cold Spring Harbor, NY: Cold Spring Harbor Laboratory Press.
Mu, W., Chu, F., Xing, Q., Yu, S., Zhou, L., & Jiang, B. (2011). Cloning, Expression, and Characterization of a D-Psicose 3-Epimerase from Clostridium cellulolyticum H10. Journal of Agricultural and Food Chemistry, 59(14), 7785-7792. https://doi.org/10.1021/jf201356q
Chan, H.-C., Zhu, Y., Hu, Y., Ko, T.-P., Huang, C.-H., Ren, F., Chen, C.-C., Ma, Y., Guo, R.-T., & Sun, Y. (2012). Crystal structures of D-psicose 3-epimerase from Clostridium cellulolyticum H10 and its complex with ketohexose sugars. Protein & Cell, 3(2), 123-131. https://doi.org/10.1007/s13238-012-2026-5
Protein [Internet]. Bethesda (MD): National Library of Medicine (US), National Center for Biotechnology Information. Accession No. WP_063903495.1, SDR family oxidoreductase [Gluconobacter frateurii]. Verfügbar unter: https://www.ncbi.nlm.nih.gov/protein/WP_063903495.1 (Zugriff am 18.12.2023)
Protein [Internet]. Bethesda (MD): National Library of Medicine (US), National Center for Biotechnology Information. Accession No. WP_265716617.1, SDR family oxidoreductase [Klebsiella pneumoniae]. Verfügbar unter: https://www.ncbi.nlm.nih.gov/protein/WP_265716617.1 (Zugriff am 18.12.2023)
Sakoda, H., & Imanaka, T. (1992). Cloning and sequencing of the gene coding for alcohol dehydrogenase of Bacillus stearothermophilus and rational shift of the optimum pH. Journal of Bacteriology, 174(4), 1397-1402. https://doi.org/10.1128/jb.174.4.1397-1402.1992
Protein [Internet]. Bethesda (MD): National Library of Medicine (US), National Center for Biotechnology Information. Accession No. WP_033015595.1, MULTISPECIES: alcohol dehydrogenase AdhP [Geobacillus]. Verfügbar unter: https://www.ncbi.nlm.nih.gov/protein/WP_033015595.1/ (Zugriff am 18.12.2023)
Protein [Internet]. Bethesda (MD): National Library of Medicine (US), National Center for Biotechnology Information. Accession No. MDQ0804260.1, Glucose 1-dehydrogenase [Priestia megaterium]. Verfügbar unter: https://www.ncbi.nlm.nih.gov/protein/MDQ0804260.1 (Zugriff am 18.12.2023)

## Patentansprüche

1. Verfahren zur Herstellung von Allitol, indem aus D-Fructose, welche in einer wässerigen Lösung vorliegt, durch Behandeln mit einer Epimerase *in vitro* D-Psicose gebildet wird, die durch Behandeln mit einer NAD(P)H-abhängigen Oxidoreduktase *in vitro* zu Allitol reduziert wird, wobei das bei der Reduktion entstandene NAD(P)⁺ mit einem Wasserstoff-Donor enzymatisch wieder zu NAD(P)H reduziert wird,
**dadurch gekennzeichnet,**
**dass** als Wasserstoff-Donor ein sekundärer Alkohol eingesetzt wird.

2. Verfahren nach Anspruch 1, **dadurch gekennzeichnet, dass** der sekundäre Alkohol 2-Propanol ist.

3. Verfahren nach Anspruch 1, **dadurch gekennzeichnet, dass** der sekundäre Alkohol D-Glucose ist.

4. Verfahren nach einem der Ansprüche 1 bis 3, **dadurch gekennzeichnet, dass** es als Eintopf-Reaktion ohne Isolierung von Zwischenprodukten durchgeführt wird.

5. Verfahren nach einem der Ansprüche 1 bis 4, **dadurch gekennzeichnet, dass** die Enzyme als Lysat der entsprechenden, sie bildenden Zellen vorliegen.

6. Verfahren nach einem der Ansprüche 1 bis 5, **dadurch gekennzeichnet, dass** die NAD(P)H-abhängige Oxidoreduktase zur Reduktion von D-Psicose zu Allitol eine Aminosäuresequenz umfasst, welche ausgewählt ist aus der Gruppe bestehend aus:
i) einer Aminosäuresequenz, die eine Identität zu SEQ ID Nr. 2 von mindestens 80% aufweist,
ii) einer Aminosäuresequenz, die durch eine Nukleinsäure kodiert wird, die eine Identität zu SEQ ID Nr. 1 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 1 bindet.

7. Verfahren nach einem der Ansprüche 1 bis 6, **dadurch gekennzeichnet, dass** zur enzymatischen Reduktion von NAD(P)⁺ eine Alkoholdehydrogenase eingesetzt wird, die vorzugsweise eine Aminosäuresequenz umfasst, welche ausgewählt ist aus der Gruppe bestehend aus:
i) einer Aminosäuresequenz, die eine Identität zu SEQ ID Nr. 4 von mindestens 80% aufweist,
ii) einer Aminosäuresequenz, die durch eine Nukleinsäure kodiert wird, die eine Identität zu SEQ ID Nr. 3 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 3 bindet.

8. Verfahren nach einem der Ansprüche 1 bis 6, **dadurch gekennzeichnet, dass** zur enzymatischen Reduktion von NAD(P)⁺ eine Glucose-Dehydrogenase eingesetzt wird, die vorzugsweise eine Aminosäuresequenz umfasst, welche ausgewählt ist aus der Gruppe bestehend aus:
i) einer Aminosäuresequenz, die eine Identität zu SEQ ID Nr. 6 von mindestens 80% aufweist,
ii) einer Aminosäuresequenz, die durch eine Nukleinsäure kodiert wird, die eine Identität zu SEQ ID Nr. 5 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 5 bindet.

9. Verwendung einer Oxidoreduktase zur Reduktion von D-Psicose zu Allitol, **dadurch gekennzeichnet, dass** die Oxidoreduktase eine Aminosäuresequenz umfasst, welche ausgewählt ist aus der Gruppe bestehend aus:
i) einer Aminosäuresequenz, die eine Identität zu SEQ ID Nr. 2 von mindestens 80% aufweist,
ii) einer Aminosäuresequenz, die durch eine Nukleinsäure kodiert wird, die eine Identität zu SEQ ID Nr. 2 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 1 bindet.
